# EUROPEAN PATENT APPLICATION

(11) **EP 3 933 407 A1**
(43) Date of publication of application: **05.01.2022**
(21) Application number: 20183960.2
(22) Date of filing: 03.07.2020
(51) Int. Cl.: G01N 33/569, A61K 39/00

(54) **METHOD FOR THE DETECTION AND MONITORING OF CAR-T-CELLS**

(71) Applicant: Johann Wolfgang Goethe-Universität Frankfurt, 60323 Frankfurt am Main (DE)
(72) Inventor: Ullrich, Evelyn, 79110 Freiburg (DE); Müller, Stephan, 63505 Langenselbold (DE)
(74) Representative: Krauss, Jan

(57) **Abstract**

The present invention relates to an improved method for the normalized detection of antigen-specific chimeric antigen receptor (CAR)-T-cells in a sample obtained from at least one patient receiving or having received a CAR-T-cell therapy specific for said antigen, as well as kits and respective uses of this method in CAR-T-cell therapy.

## Description

The present invention relates to an improved method for the normalized detection of antigen-specific chimeric antigen receptor (CAR)-T-cells in a sample obtained from at least one patient receiving or having received a CAR-T-cell therapy specific for said antigen, as well as kits and respective uses of this method in CAR-T-cell therapy.

### Background of the invention

Chimeric antigen receptor T cell (CAR-T) therapies have now entered mainstream clinical practice with two approved autologous CAR-T products targeting CD19 and numerous other products in early and late phase clinical trials.

The first two FDA-approved CAR-T therapies both target the CD19 antigen, which is found on many types of B-cell cancers. Tisagenlecleucel (Kymriah / Novartis) is approved to treat relapsed/refractory B-cell precursor acute lymphoblastic leukemia (ALL), while axicabtagene ciloleucel (Yescarta / Kite Pharma) is approved to treat relapsed/refractory diffuse large B-cell lymphoma (DLBCL). Thus, anti-CD 19-CAR is currently clinically available as one of the therapeutic modalities for refractory acute B-cell-typed lymphoblastic leukemia (B-ALL) patients.

This has led to a demand for highly sensitive, specific, and easily reproducible methods to monitor CAR-T cells in patients. In order to detect CAR on the cell surface and investigate the efficacy of CAR-T cells, there are numerous experimental approaches, including flow cytometry, Cr-releasing assay, immunoblot, and immunostaining.

Detection of CAR-bearing cells is usually performed by flow cytometry, with the use of antibodies against the extracellular structure of the molecule, such as the hinge (using an anti-IgG Fc antibody or F(ab')2 fragment) or the antigen-binding domains (as in the case of the use of an anti-idiotypic antibody).

Jozwik A., et al. (in: Monitoring Allogeneic CAR-T Cells Using Flow Cytometry. In: Katz S., Rabinovich P. (eds) Cell Reprogramming for Immunotherapy. Methods in Molecular Biology, vol 2097. Humana, New York, NY (2020)) describe a flow cytometry based protocol for detection of allogeneic CAR-T cells and for monitoring their phenotype and numbers in blood and bone marrow of patients following CAR-T treatment.

Mihara K., et al. (in: Basic Procedures for Detection and Cytotoxicity of Chimeric Antigen Receptors. In: Steinitz M. (eds) Human Monoclonal Antibodies. Methods in Molecular Biology, vol 1904. Humana Press, New York, NY (2019)) describe protocols for basic experiments and procedures for the detection of CAR on transduced cells and in in vitro coculture experiments to assess cytotoxicity using CAR-T cells.

Zheng, Z., et al. (in: Protein L: a novel reagent for the detection of Chimeric Antigen Receptor (CAR) expression by flow cytometry. J Transl Med 10, 29 (2012) https://doi.org/10.1186/1479-5876-10-29) used CARs derived from both human and murine antibodies to validate this novel protein L based flow cytometric method and the results correlated well with other established methods. Activated human PBLs were transduced with retroviral vectors expressing two human antibody based CARs (anti-EGFRvIII, and anti-VEGFR2), two murine antibody derived CARs (anti-CSPG4, and anti-CD 19), and two humanized mouse antibody based CARs (anti-ERBB2, and anti-PSCA). Transduced cells were stained first with biotin labeled protein L followed by phycoerythrin (PE)-conjugated streptavidin (SA) and analyzed by flow cytometry.

De Oliveira, S.N., et al. (in: A CD19/Fc fusion protein for detection of anti-CD19 chimeric antigen receptors. J Transl Med 11, 23 (2013). https://doi.org/10.1186/1479-5876-11-23) developed a CD19/Fc molecule that can be labeled for its use primarily as a reagent in flow cytometry studies. The approach consisted of fusing the extracellular domains of the human CD19 protein to the human immunoglobulin Fc domain. Fusion to the Fc domain has been used to allow secretion of peptide sequences, with enhanced solubility and stability, and a fusion protein of murine extracellular CD19 and Fc domain has been previously described. They describe the studies for the development and evaluation of this fusion protein. The properties of this reagent make possible sensitive detection by flow cytometry of cells modified with CD19-specific CAR

Jena B, et al. (in: Chimeric Antigen Receptor (CAR)-Specific Monoclonal Antibody to Detect CD19-Specific T Cells in Clinical Trials. PLoS ONE 8(3): e57838. (2013) https://doi.org/10.1371/journal.pone.0057838) describe a novel anti-idiotype monoclonal antibody (mAb) to detect CD19-specific CAR+ T cells before and after their adoptive transfer. The CD19-specific CAR mAb is described as useful to investigators implementing CD19-specific CAR+ T cells to treat B-lineage malignancies. See also US20180265595A1.

The detection of circulating CD19-CAR-T-cells in patients that undergo therapy usually involves standardized polymerase-chain reaction (PCR), nevertheless, this is done mostly in specialized clinical facilities. Standardized and methods that are easy to establish and are reproducible for a detection of CD19-CAR-T-cells to be used in any laboratory, ideally at the point of care or in the context of CD19-CAR-T-studies, are missing. Despite the improvements in the design of CARs and expansion of the number of target antigens, there is also no universal flow cytometric method available to detect the expression of CARs on the surface of transduced lymphocytes.

It is therefore an object of the present invention to provide such a standardized and robust method for the detection of circulating CD19-CAR-T-cells in patients. Other objects and advantages will become apparent to the person of skill when studying the present description of the present invention.

In a first aspect of the present invention, this object is solved by an improved method for the normalized detection of antigen-specific chimeric antigen receptor (CAR)-immune cells, such as T-cells, in a sample obtained from at least one patient receiving or having received a CAR-immune-cell therapy specific for said antigen, said method comprising the steps of a) staining of leukocytes in a suitable sample obtained from said at least one patient using anti-antigen- and anti-CD3-antibody, and b) detection of antigen-CAR-immune-cells comprising
i) a first detection of said antigen-CAR-immune-cells of a) comprising fluorescence-activated cell sorting (FACS), and ii) a second detection of antigen-CAR-immune-cells in a cell-based reference system comprising FACS, wherein said cell-based reference system comprises, as a positive control, antigen-CAR-expressing reference cells, such as, for example, antigen-CAR HEK-293 T cells, additionally expressing a recombinant cell-surface marker tag, and leukocytes obtained from a healthy subject as a negative control; and c) normalizing said detection of said antigen-CAR-immune-cells in said sample obtained from said patient using the values as obtained with said first and second detection, whereby the normalized detection of antigen-specific chimeric antigen receptor (CAR)-immune-cells in said sample is achieved. Preferably, said immune cells are selected from T cells, CIK cells, gdT cells, NKT cells, and others. Preferably, said method is performed to accompany said CAR-T therapy.

In the context of the invention, all suitably antigen-CAR expressing cells, either cell lines or immune cell populations, can be used. These are designated "antigen-CAR-expressing reference cells".

In a second aspect of the present invention, this object is solved by a method for identifying a compound that improves a CAR-immune-cell therapy in a patient undergoing such therapy, comprising a) administering at least one test compound to said patient in combination with said CAR-immune cell therapy, b) performing the normalized detection of CAR-immune-cells according to the method of the invention in a sample obtained from said patient, c) comparing said CAR-immune-cells as detected with a control sample in the absence of said at least one compound, and d) identifying said at least one compound as improving said CAR-immune-cell therapy in said patient, if the number of CAR-immune-cells is about identical or increased in said sample obtained from said patient when compared to said control.

A third aspect of the invention relates to a kit for the normalized detection of antigen-specific chimeric antigen receptor (CAR)-immune-cells in a sample, comprising materials for performing the method according to the present invention, in particular materials selected from a cell line comprising a recombinant cell-surface marker tag, anti-antigen- and/or anti-CD3-antibodies, FITC-labelled antigen, plasmids encoding an antigen-CAR, buffers, and labelling chemistry. Another aspect is the use of the kit according to the present invention for the normalized detection of CAR-immune-cells in a sample according to a method according to the present invention.

In a fourth aspect thereof, the present invention relates to a method for treating a viral infection or cancer, comprising performing the method according to according to the present invention on a sample obtained from a patient undergoing or having undergone a CAR-immune-cell treatment against said viral infection or cancer, and suitably adjusting said treatment based on the number of CAR-immune-cells as identified.

For the above aspects, preferably, said immune cells are selected from T cells, CIK cells, gdT cells, NKT cells, and others.

The improved method for the normalized detection of antigen-specific chimeric antigen receptor (CAR)-T-cells in a sample obtained from at least one patient receiving or having received a CAR-T-cell therapy specific for said antigen comprises the steps of a) staining of leukocytes in a suitable sample obtained from said at least one patient using anti-antigen- and anti-CD3-antibody, and b) detection of antigen-CAR-T-cells comprising i) a first detection of said antigen-CAR-T-cells of a) comprising fluorescence-activated cell sorting (FACS), and ii) a second detection of antigen-CAR-T-cells in a cell-based reference system comprising FACS, wherein said cell-based reference system comprises, as a positive control, antigen-CAR-T-cells additionally expressing a recombinant cell-surface marker tag, and leukocytes obtained from a healthy subject as a negative control; and c) normalizing said detection of said antigen-CAR-T-cells in said sample obtained from said patient using the values as obtained with said first and second detection, whereby the normalized detection of antigen-specific chimeric antigen receptor (CAR)-T-cells in said sample is achieved. Preferably, said method is performed to accompany said CAR-T therapy.

In the context of the present invention, "staining" usually relates to a process where an antibody or antibody binding domain construct binds to its epitope(s), thereby providing, directly or indirectly, a signal that can be detected and/or attaching a group that allows selection and/or identification of said epitope carrying entity. In the context of the invention, staining is preferably fluorescent, and is used for FACS.

In the context of the present invention, a patient can be a mammalian patient, preferably a human.

Commonly, a detection of CAR-expressing T cells, such as CD19-CAR-expressing T cells, in samples, such as peripheral blood, of patients treated with said (CD19-)CAR-T cell therapy/infusion is performed with standardized PCR-based methods. These methods are laborious and error-prone, and therefore routinely performed only by those centers that essentially participate in the development of this new therapy form.

The present approach of a flow cytometry-based CAR detection assay offers a standardized (normalized) method that can be easily established in most laboratories. With the use of an antigen specific, e.g. CD19-specific, human protein, the present method is more precise in detecting antigen CAR expressing cells than previous approaches using flow cytometry based on detection methods with more unspecific binding reagents, such as Protein-L and anti-IgG Fc antibodies or F(ab')2 fragment antibodies (see, for example, Zheng Z, Chinnasamy N, Morgan RA. Protein L: a novel reagent for the detection of chimeric antigen receptor (CAR) expression by flow cytometry. J Transl Med. 2012;10:29. doi:10.1186/1479-5876-10-29. Kochenderfer JN, Feldman SA, Zhao Y, et al. Construction and preclinical evaluation of an anti-CD19 chimeric antigen receptor. J Immunother. 2009;32(7):689-7021, 2).

In addition, with the use of commercially available reagents, previous approaches using self-made fusion-proteins or self-made CD19-CAR specific antibodies are outdated (see, for example, Satiro N De Oliveira, Jiexin Wang, Christine Ryan, Sherie L Morrison, Donald B Kohn, Roger P Hollis. A CD19/Fc fusion protein for detection of anti-CD19 chimeric antigen receptors. Jena B, Maiti S, Huls H, et al. Chimeric antigen receptor (CAR)-specific monoclonal antibody to detect CD19-specific T cells in clinical trials. PLoS ONE. 2013;8(3):e57838. doi:10.1371/journal.pone.00578383). Several companies (e.g. Miltenyibiotec, Acrobiosystems, ThermoFisherScientific) have developed commercially available (CD19-)CAR detection reagents and antibodies in addition to the FITC-CD19 protein as used in the present examples.

In general, any suitable sample can be used for the methods according to the invention. The sample has to contain leukocytes, and in particular the CAR-T carrying cells, such as T-cells or recombinantly transfected cells. In case a peripheral blood sample is used, the method according to the present invention preferably comprises the steps of treating the blood sample from said patient with a suitable anticoagulant, such as EDTA, followed by a suitable lysis or other removal of erythrocytes before the detection of leukocytes, i.e. the T-cells. The person of skill is well aware of suitable reagents to avoid coagulation, and to remove potentially interfering components (e.g. the erythrocytes using the ACK buffer as described herein).

In one particular embodiment, peripheral blood was taken from patients that underwent therapy using CD19-CAR-T-cells (e.g. Kymriah), and was transferred into EDTA collection tubes. Erythrocytes were lysed using an "ammonium-chloride-potassium" (ACK) buffer, and the leukocytes were separated from the other blood components using centrifugation. Then, these leukocytes, including the CD19-CAR-T-cells, are stained with a suitably labelled (e.g. fluorescently labelled) CD19 protein (Acrobiosystems), and a suitably labelled (e.g. fluorescently labelled) anti-CD3 antibody in order to discriminate CD19-CAR-T-cells using flow cytometry. CD19-CAR positive HEK-cells (= positive control) are used as standardized control of the staining using the CD19 protein, and blood samples of healthy subjects are used as negative control. For this positive control CD19-CAR positive HEK-cells were thawed and stained with the CD19 protein. Another sample (aliquot) of the HEK-cells was stained with an anti-myc antibody that specifically binds to the myc sequence of the CD19-CAR of the HEK-cells. As an advantage and for safety reasons, such a myc sequence is no longer contained in the CD19-CAR-T-cells as used for the therapy.

In a preferred embodiment of the method according to the present invention, the method further comprises the step of determining the number of cells (the cell count) as detected. Cell counts can be determined using standard methods, such as by using a Neubauer chamber.

As mentioned above, the method according to the present invention comprises a staining of leukocytes (and also cells of the reference system) using respective antibodies. These antibodies can be full antibodies or antigen binding parts thereof (e.g. scFvs), and also include bispecific or multispecific constructs, such as an anti-antigen-and anti-CD3-antibody bispecific. Preferred are CD19 and CD3 binders.

Alternatively to the CD19 assay as described here as an example, the present assay comprising the quality control measures (reference system, normalization system) can be easily adapted for a variety of other specific CARs, besides the CD19-specific CAR Preferably, the antigen can be selected from CD30, CD33, CD123, FLT3, BCMA, CD20, CD22, ROR1, κ light chain, CD133, CD138, and CD19.

An essential component of the present method is the use of the cell-based reference system that comprises, as a positive control, antigen CAR-expressing reference cells, such as, for example, antigen-CAR-T-cells additionally expressing a recombinant cell-surface marker tag. Furthermore, leukocytes obtained from a healthy subject can be used as a negative control, as it is expected that this sample does not comprise antigen-CAR-T-cells. The system is then used for a "quality control" of the assay, e.g. by normalizing said detection of said antigen-CAR-T-cells in said sample obtained from said patient using the values (results) as obtained with said first and second detection. Thereby, the normalized detection of antigen-specific chimeric antigen receptor (CAR)-T-cells in said sample is achieved. The reference system is cell-based, and preferably the cells of the reference system cells are selected from mammalian, such as human cell lines, such as, for example, CHO, HEK, HeLa or autologous or heterologous T-cells. Preferred are human cell lines.

In the reference system, recombinant cells are used that express the antigen-CAR in addition to a (preferably recombinant) cell-surface marker tag. This tag can be stained, and is thus preferably selected from an antigenic polypeptide, such as, for example, a myc-tag, FLAG tag, ALFA-tag, V5-tag, HA-tag, Spot-tag, T7-tag, and NE-tag.

While the method according to the present invention can be performed once using a sample (or pool of samples) from a patient (or patients) undergoing or that have undergone CAR-T cell therapy, in one embodiment, the method further comprises a monitoring of said antigen-CAR-T-cells as detected in said patient over time. That is, the number and/or quality of CAR-T cells is followed over time using several samples of the same patient, or even a group of patients. The values are compared to a control, and preferably said sample and/or the control is/are obtained from one patient or subject, or a group or pool of patients or subjects, depending on the circumstances of the detection.

In another preferred aspect of the method according to the present invention, the method is performed to accompany said CAR-T therapy, to determine its applicability to a specific person, and optimally to determine responders and non-responders to the therapy.

Yet another aspect of the present invention relates to a method for identifying a compound that improves a CAR-T-cell therapy in a patient undergoing such therapy, comprising a) administering at least one test compound to said patient in combination with said CAR-T-cell therapy, b) performing the normalized detection of CAR-T-cells according to the method according to the present invention in a sample obtained from said patient, c) comparing said CAR-T-cells as detected with a control sample in the absence of said at least one compound, and d) identifying said at least one compound as improving said CAR-T-cell therapy in said patient, if the number of CAR-T-cells is about identical or increased in said sample obtained from said patient when compared to said control.

Within this aspect, the present method can be employed to develop compounds that can be used in the context of a CAR-T-cell therapy. These therapeutic molecules can be of a wide variety of compound, such as selected from a proteinaceous domain, a small molecule (less than 500 kDa), a peptide, antibodies, an environmental substance, probiotic, toxin, aerosol, medicine, nutrient, galenic composition, plant extract, volatile compound, homeopathic substance, incense, pharmaceutical drug, vaccine, a compound or compound mixture derived from organisms, for example animals, plants, fungi, bacteria, archaea, a chemical compound, a compound used in food or cosmetic industry, and a library of said compounds.

Yet another aspect of the present invention then relates to a diagnostic kit comprising materials for performing a method according to the present invention in one or separate containers, optionally together with auxiliary agents and/or instructions for performing said method. Preferred is a kit for the normalized detection of antigen-specific chimeric antigen receptor (CAR)-T-cells in a sample, comprising materials for performing the method according to the present invention, in particular materials selected from a cell line comprising a recombinant cell-surface marker tag, anti-antigen- and/or anti-CD3-antibodies, FITC-labelled antigen, plasmids encoding an antigen-CAR, buffers, and labelling chemistry. The kit may comprise relevant antibodies, recombinant marker protein, respective peptides, dyes and other labels, as well buffers and matrices for performing the tests.

Preferred is the use of said diagnostic kit according to the present invention for a method according to the present invention, i.e. for the normalized detection of CAR-T-cells in a sample according to a method according to the present invention.

Yet another aspect of the present invention then relates to a method for treating a viral infection or cancer, comprising performing the method according to the present invention on a sample obtained from a patient undergoing or having undergone a CAR-T-cell treatment against said viral infection or cancer, and suitably adjusting said treatment based on the number of CAR-T-cells as identified. Here, the method is used to select new or improved treatment options, based on their effect(s) on the CAR-T-cells and their statuses/numbers as analyzed.

The present inventors see further possible embodiments for the present method in the detection of various newly developed CARs against a diverse range of antigens, in the detection of CAR expression on all types of immune cells, in combination of CAR detection with immune status evaluation as a companion diagnostic or a panel ready for use, and in development of kits for CAR detection and immune monitoring.

The reference system according to the invention consists of CD19-CAR positive HEK-cells that additionally express a "myc-tag" as a positive control, and blood samples of healthy subjects as negative control. According to the invention, one part of the CD19-CAR positive HEK-cells is stained with CD-19-antibody and one part with anti-mycantibody. For safety reasons, a myc- (or different) sequence is no longer contained in the CD19-CAR-T-cells that are used for therapy.

The present method was validated using samples from 18 patients, and monitoring was performed for more than 100 days. In both scenarios, CAR-T-cells were reliably identified and detected.

The present method can be quickly established, offers pre-clinical quality measures and a standardized ready to use assay for the measurement of patient blood.

The present invention will now be described further in the following examples with reference to the accompanying Figures, nevertheless, without being limited thereto. For the purposes of the present invention, all references as cited herein are incorporated by reference in their entireties.

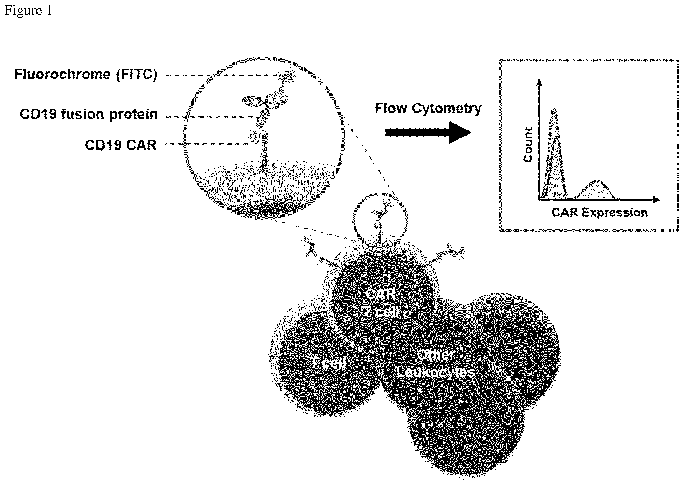

**Figure 1** shows the principle of detection of CD19-CAR-expressing T cells within blood samples of patients treated with CD19-CAR-T cell infusions after erythrocytes were lysed and flow cytometry-based detection of CD19-CAR-expressing T cells was performed in remaining leukocytes (CD19-CAR-T cells depicted in turquoise with CAR-expression, T cells depicted in turquoise and other leukocytes depicted in blue). The FITC-labeled-CD19 protein binds the CD19-CAR on expressing T cells.

Afterwards the CAR-expressing T cells determined as FITC-positive signal were analyzed using histograms.

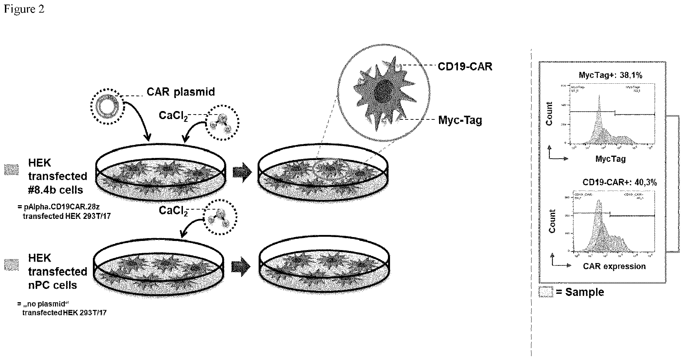

**Figure 2** shows the development of the reference cell system using the HEK cell line. HEK cells were transfected with a plasmid containing sequences for the CD19-CAR receptor and the polypeptide protein tag "Myc tag" using a calcium phosphate-based transfection procedure generating HEK transfected #8.4b cells. As negative controls to these cells, HEK cells were used, which received the same calcium phosphate-based transfection procedure without using a plasmid, generating HEK transfected nPC cells. Both were stained with the FITC-labeled-CD19-protein and the PE conjugated Myc tag specific antibody and were measured at a flow cytometer. Analysis was performed using histograms (red areas represent HEK transfected #8.4b cells, blue areas represent HEK transfected nPC cells).

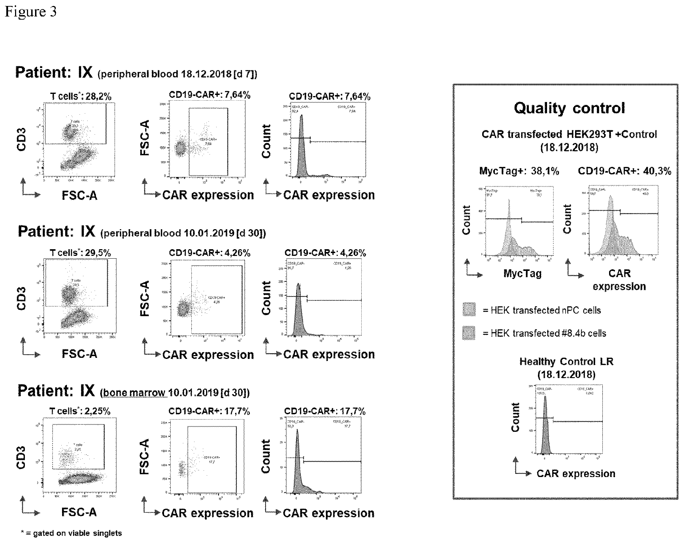

**Figure 3** shows an example of detection of CD19-CAR-T cells in peripheral blood and bone marrow of a patient treated with CD 19-CAR-T cell infusions. Measurements were performed 7 (peripheral blood) and 30 (peripheral blood and bone marrow) days after infusion. Quality controls (depicted on the right) were performed with the reference cell system and peripheral blood of a healthy person.

### Examples

With respect to the examples as described herein, generally also other suitable markers, such as fluorochromes, FACS instruments, and software can be used for assays according to the present invention, and the person of skill can readily modify the methods accordingly. Furthermore, the exemplary antigen-specific fluorochromelabeled human CD19 (20-291) protein, Fc Tag can be replaced by any other suitable CAR-specific protein, such as CD30 (in refractory Hodgkin's lymphoma); CD33, CD123, and FLT3 (in acute myeloid leukemia (AML)); BCMA (in multiple myeloma);

CD20, CD22, ROR1, κ light chain, CD133, and CD138; and the CAR then has the respective specificity.

### Detection of CD19-CAR-expressing T cells

To detect CD19-CAR-expressing T cells in peripheral blood of patients treated with CD19-CAR-T cell infusions, for example Kymriah (Novartis) the following CD19-CAR detection assay was developed, using a FITC-labeled human CD19 (20-291) Protein, Fc Tag (FITC-labeled-CD19 protein) (Acro Biosystems).

2ml of EDTA whole blood samples from patients that have been treated with CD19-CAR-T cells were diluted with 40ml of 37°C pre-warmed ACK (Ammonium-Chloride-Potassium) lysis buffer and centrifuged at 400 × g for 7 minutes. After centrifugation, the supernatant was discarded, and the cell pellet was resuspended with 4ml PBS (Phosphate-Buffered Saline). The suspension was once more centrifuged at 400 × g for 7 minutes, the supernatant was discarded, and the cells were resuspended in an appropriate volume of RPMI (Roswell Park Memorial Institute) 1640 medium, GlutaMAX supplemented with 10% fetal bovine serum (FBS) and 1% penicillin/streptomycin (Pen/Strep).

The cell count was estimated with a Neubauer chamber. Afterwards, 0.2 × 10⁶ cells were distributed to FACS tubes and washed with 1 ml PBS + 2% bovine serum albumin (BSA) at 400 × g for 7 minutes. After the supernatant was discarded, the cells were stained in the following order with FITC-labeled-CD19 protein, CD3 BUV395 (BD Biosciences) and 7-AAD (BD Biosciences).

First, 100 µl of the FITC-labeled-CD19 protein working solution (100 µg/ml of FITC-Labeled human CD19 (20-291) Protein, Fc Tag diluted at 1:10 with PBS + 2% BSA) was added to the cells. After an incubation period for 1 hour at 4°C in the dark, the cells were washed three times with 2 ml PBS + 2% BSA at 400 × g for 7 minutes and the respective supernatant was discarded. Following the last washing step, the cells were resuspended in 100 µl FACS buffer (CellWASH + 0.5% BSA + 0.05% NaN₃) and staining with 2.5 µl CD3 BUV395 antibody was performed. After an incubation for 20 minutes at 4°C in the dark, the cells were washed with 1 ml PBS + 2% BSA at 300 × g for 5 minutes. The supernatant was discarded, and the cells were resuspended in 100 µl FACS buffer. Finally, cells were stained with 2.5 µl 7-AAD to determine dead cells. After a last incubation at 4°C for 10 minutes in the dark, another 100 µl FACS buffer was added and the samples were measured at a BD FACSCelesta flow cytometer (BD Biosciences). As negative controls blood of healthy donors was used. Data analysis was performed with FlowJo (FlowJo LLC).

### Reference assay for the CD19-CAR detection assay using HEK cells

As a control/reference for the procedure, a CD19-CAR expressing HEK cell line was generated. For this purpose, HEK cells were transfected with a plasmid containing sequences for the CD19-CAR receptor and the polypeptide protein tag "Myc tag" (HEK transfected #8.4b cells). Three separate stains of HEK transfected #8.4b cells were performed: One with the FITC-labeled-CD19 protein, one with a PE conjugated Myc tag specific antibody (Cell Signaling Technology) and the last one with 7-AAD. These controls were performed to ensure that the FITC-labeled-CD19 protein was specifically binding the CD19-CAR receptor. As negative controls, HEK cells were used, which received the same calcium phosphate-based transfection procedure as HEK transfected #8.4b cells, with the difference that no plasmid was added (in the following referred to as HEK transfected nPC cells).

HEK cells (HEK transfected #8.4b and HEK transfected nPC cells) frozen at -80°C were thawed. Therefore, they were warmed in a 37°C water bath for a few seconds until the ice began to melt. Afterwards, the cell suspension was emptied in 30 ml of pre-warmed Dulbecco's Modified Eagle Medium (DMEM) high glucose supplemented with 10% FBS and 1% L-Glutamine (all Gibco). The suspension was centrifuged at 400 × g for 7 minutes, the supernatant was discarded, and the cell pellet was resuspended in appropriate volume of DMEM high glucose medium supplemented with 10% FBS and 1% L-Glutamine.

Cells were then counted with a Neubauer chamber, and 0.2 × 10⁶ cells were distributed to FACS tubes. The cells were washed once with 1 ml PBS + 2% BSA at 400 × g for 7 minutes. After the supernatant was discarded, staining of the cells was performed.

For staining with FITC-labeled-CD19 protein, 100 µl of the working solution (see above) was added to the cells. After an incubation for 1 hour at 4°C in the dark the cells were washed three times with 2 ml PBS + 2% BSA at 400 × g for 7 minutes and the respective supernatant was discarded. Afterwards, cells were resuspended in 200 µl FACS buffer and were ready for measurement. For staining with the Myc tag antibody 1 µl of the antibody in 100 µl FACS buffer was added to the cells. After an incubation period for 30 minutes at 4°C in the dark, the cells were once washed with 1 ml PBS + 2% BSA at 300 × g for 5 minutes and the supernatant was discarded. At last the cells were resuspended in 200 µl FACS buffer and were ready for measurement. For 7-AAD stained controls, 2.5 µl of 7-AAD in 100 µl FACS buffer was added to the cells. After an incubation period of 10 minutes at 4°C in the dark, 100 µl FACS buffer were added and the cells were ready for measurement. Cells were measured at the BD FACSCelesta flow cytometer and data analysis was performed with FlowJo.

## Claims

1. An improved method for the normalized detection of antigen-specific chimeric antigen receptor (CAR) immune-cells, such as T cells, CIK cells, gdT cells, NKT cells and others in a sample obtained from at least one patient receiving or having received a CAR-T-cell therapy specific for said antigen, said method comprising the steps of
a) staining of leukocytes in a suitable sample obtained from said at least one patient using anti-antigen- and, optionally, anti-immune cell-, such as CD3-, antibody, and
b) detection of antigen-CAR-immune-cells comprising
i) a first detection of said antigen-CAR-immune-cells of a) comprising fluorescence-activated cell sorting (FACS), and
ii) a second detection of antigen-CAR-immune-cells in a cell-based reference system comprising FACS, wherein said cell-based reference system comprises, as a positive control, antigen-CAR-immune-cells additionally expressing a recombinant cell-surface marker tag, and leukocytes obtained from a healthy subject as a negative control; and
c) normalizing said detection of said antigen-CAR-immune-cells in said sample obtained from said patient using the values as obtained with said first and second detection, whereby the normalized detection of antigen-specific chimeric antigen receptor (CAR)-immune-cells in said sample is achieved.

2. The method according to claim 1, wherein said method further comprises the steps of obtaining a blood sample from said patient, treating said sample with a suitable anticoagulant, and suitable lysis of erythrocytes before a detection of leukocytes.

3. The method according to claim 1 or 2, wherein said method further comprises the step of determining the number of cells as detected.

4. The method according to any one of claims 1 to 3, wherein said staining of leukocytes comprises a bispecific or multispecific anti-antigen- and anti-immune cell, such as CD3-, antibody.

5. The method according to any one of claims 1 to 4, wherein said antigen is selected from CD30, CD33, CD123, FLT3, BCMA, CD20, CD22, ROR1, κ light chain, CD133, CD138, and CD19.

6. The method according to any one of claims 1 to 5, wherein said recombinant cell-surface marker tag is selected from an antigenic polypeptide, such as, for example, a myc-tag, FLAG tag, ALFA-tag, V5-tag, HA-tag, Spot-tag, T7-tag, and NE-tag.

7. The method according to any one of claims 1 to 6, wherein said reference system cells are selected from mammalian, such as human cell lines, such as, for example, CHO, HEK, HeLa or autologous or heterologous T-cells..

8. The method according to any one of claims 1 to 7, wherein said method further comprises monitoring of said antigen-CAR-immune-cells as detected in said patient over time.

9. The method according to any one of claims 1 to 8, wherein said sample and/or the control is/are obtained from one patient or subject, or a group or pool of patients or subj ects.

10. The method according to any one of claims 1 to 9, wherein said method is performed to accompany said CAR-T therapy.

11. A method for identifying a compound that improves a CAR-immune-cell therapy in a patient undergoing such therapy, comprising
a) administering at least one test compound to said patient in combination with said CAR-T-cell therapy,
b) performing the normalized detection of CAR-immune-cells according to the method of any one of claims 1 to 10 in a sample obtained from said patient,
c) comparing said CAR-immune-cells as detected with a control sample in the absence of said at least one compound, and
d) identifying said at least one compound as improving said CAR-immune-cell therapy in said patient, if the number of CAR-immune-cells is about identical or increased in said sample obtained from said patient when compared to said control.

12. The method according to claim 11, wherein said compound is selected from a proteinaceous domain, a small molecule, a peptide, antibodies, an environmental substance, probiotic, toxin, aerosol, medicine, nutrient, galenic composition, plant extract, volatile compound, homeopathic substance, incense, pharmaceutical drug, vaccine, a compound or compound mixture derived from organisms, for example animals, plants, fungi, bacteria, archaea, a chemical compound, a compound used in food or cosmetic industry, and a library of said compounds.

13. A kit for the normalized detection of antigen-specific chimeric antigen receptor (CAR)-immune-cells in a sample, comprising materials for performing the method according to any one of claims 1 to 10, in particular materials selected from a cell line comprising a recombinant cell-surface marker tag, anti-antigen- and/or anti-CD3-antibodies, FITC-labelled antigen, plasmids encoding an antigen-CAR, buffers, and labelling chemistry.

14. Use of the kit according to claim 13 for the normalized detection of CAR-immune-cells in a sample according to a method according to any one of claims 1 to 10.

15. A method for treating a viral infection or cancer, comprising performing the method according to any one of claims 1 to 12 on a sample obtained from a patient undergoing or having undergone a CAR-immune-cell, in particular T-cell, treatment against said viral infection or cancer, and suitably adjusting said treatment based on the number of CAR-immune-cells as identified.
